# EUROPEAN PATENT APPLICATION

(11) **EP 3 725 870 A1**
(43) Date of publication of application: **21.10.2020**
(21) Application number: 18888018.1
(22) Date of filing: 30.11.2018
(51) Int. Cl.: C12M 1/00

(54) **LIQUID DISCHARGE DEVICE, SENSOR MANUFACTURING DEVICE HAVING LIQUID DISCHARGE DEVICE, AND CELL CULTURE DEVICE HAVING LIQUID DISCHARGE DEVICE**

(30) Priority: 15.12.2017 JP 2017240222
(71) Applicant: Panasonic Intellectual Property Management Co., Ltd., Osaka-shi, Osaka 540-6207 (JP)
(72) Inventor: YAMAMOTO Kenichi, Osaka 540-6207 (JP); NAKAGAWA Tohru, Osaka 540-6207 (JP); YOSHIDA Hidehiro, Osaka 540-6207 (JP); OHTSUKA Futoshi, Osaka 540-6207 (JP)
(74) Representative: Vigand, Philippe
(86) International application number: PCT/JP2018/044172
(87) International publication number: WO 2019/116929

(57) **Abstract**

A liquid discharge device includes a nozzle from which liquid drops are discharged, a fixing part for fixing the nozzle, and a driving part generating displacement, in which the nozzle is fixed to a first holding part in the driving part and a second holding part in the fixing part.

## Description

### TECHNICAL FIELD

The present invention relates to a liquid discharge device for discharging liquid, a sensor device having the liquid discharge device and a cell culture device having the liquid discharge device. The present invention particularly relates to a liquid discharge device suitable for being used for supplying or dispensing a chemical liquid, a reagent, etc., a sensor manufacturing device having the liquid discharge device and a cell culture device having the liquid discharge device in fields of drug discovery, medical treatment, biotechnology and the like.

### BACKGROUND ART

In recent years, as a device for recording characters and images in various recording media, an ink-jet type printing device is popular and well known. The ink-jet is applied not only to printing on paper or films but also to the fields of drug discovery, medical treatment, and biotechnology. An application range of the ink-jet is widely expanding.

In particular, an attempt to supply a minute amount of liquid chemical, reagent and so on to a specimen accurately by utilizing highly precise discharge performance is proceeding in recent years.

However, it is inevitably necessary that a wetted part is free from metal as it is easily affected by metal ions in the fields of drug discovery, medical treatment, and biotechnology. Furthermore, it is necessary to surely avoid mixture of a chemical liquid/reagent that has been used and a new chemical liquid/reagent at the time of replacing the chemical liquid/reagent because it incurs not only reduction in performance but also an unexpected reaction due to mutual interaction.

However, the inside of a normal liquid discharge head is formed of a metal material, and further, it is formed by a narrow flow path or a space for fluid. Accordingly, it is difficult to wash the inside of the liquid discharge head completely; therefore, a liquid discharge head capable of replacing the wetted part easily has been devised (Patent Literature 1).

In Patent Literature 1, a removable container is provided, and the container is pressurized from the outside by a driving mechanism. According to this, a pressure inside the container is increased so as to correspond to increase of a displacement amount in the driving mechanism, and inside liquid can be discharged from a nozzle at an opening end.

### CITATION LIST

### PATENT LITERATURE

PTL 1: JP-A-2008-114569

### SUMMARY OF INVENTION

The container can be directly pressurized at the time of pressurization, however, when the pressure is reduced, it is difficult to follow reduction of the displacement amount in the driving mechanism. It is difficult to recover to an initial state at high speed as being dominated by elastic recovery properties of the container. Accordingly, it is difficult to perform discharge at a high repetition frequency.

The present invention has been made in view of the above problems, and an object thereof is to provide a liquid discharge device capable of highly performing repetition and operating at high speed while using a disposable pressure chamber, a sensor manufacturing device having the liquid discharge device and a cell culture device having the liquid discharge device.

In order to solve the above problems, there is provided a liquid discharge device including a nozzle from which liquid drops are discharged, a fixing part for fixing the nozzle, and a driving part generating displacement, in which the nozzle is fixed to a first holding part in the driving part and a second holding part in the fixing part.

There is also provided a sensor manufacturing device including the liquid discharge device, a sensor to which liquid is applied from the liquid discharge device, a detector detecting the sensor and a controller controlling the detector.

There is further provided a cell culture device including the liquid discharge device, a container to which liquid is applied from the liquid discharge device, and a stage moving the container.

Accordingly, the following ability of displacement with respect to the driving part can be increased and discharge can be performed at a high-speed repetition frequency.

Furthermore, the state in which the contact between the driving part and the holding part of the nozzle is kept can be maintained not depending on the repetition frequency; therefore, a contact surface between the driving part and the nozzle is not damaged due to an impact caused by separation and the lifetime of the nozzle can be increased. Accordingly, it is also possible to reduce a disposal loss of the nozzle.

### BRIEF DESCRIPTION OF DRAWINGS

Fig. 1A is a perspective view showing a structure of a liquid discharge device according to Embodiment 1.
Fig. 1B is a cross-sectional view showing the structure of the liquid discharge device according to Embodiment 1.
Fig. 1C is a perspective view showing a discharging part of the structure of the liquid discharge device according to Embodiment 1.
Fig. 2 is a graph showing a relationship between an amount of discharge liquid drops per unit time and a frequency of discharge in the liquid discharge device according to Embodiment 1.
Fig. 3 is a graph showing a relationship between the amount of discharge liquid drops and the number of times of discharge in the liquid discharge device according to Embodiment 1.
Fig. 4A shows a cross-sectional view and a top view showing a modification example of a nozzle according to Embodiment 1.
Fig. 4B is a cross-sectional view showing a modification example of the nozzle according to Embodiment 1.
Fig. 5 is a view showing a structure of a liquid discharge device according to Embodiment 2.
Fig. 6A is a side view of a liquid discharge device according to Embodiment 3.
Fig. 6B is a front view of an inspection unit according to Embodiment 3.
Fig. 7A is a side view of a cell culture device according to Embodiment 3.
Fig. 7B shows a plan view of a container according to Embodiment 3.
Fig. 8A is a perspective view of a liquid discharge device according to Embodiment 4.
Fig. 8B is a front view of the liquid discharge device according to Embodiment 4.
Fig. 9A is a perspective view of a liquid discharge device formed by combining a plurality of liquid discharge devices according to Embodiment 4.
Fig. 9B is a bottom view of the liquid discharge devices according to Embodiment 4.

### DESCRIPTION OF EMBODIMENTS

Hereinafter, embodiments of the present invention will be explained with reference to the drawings.

### (Embodiment 1)

### <Structure>

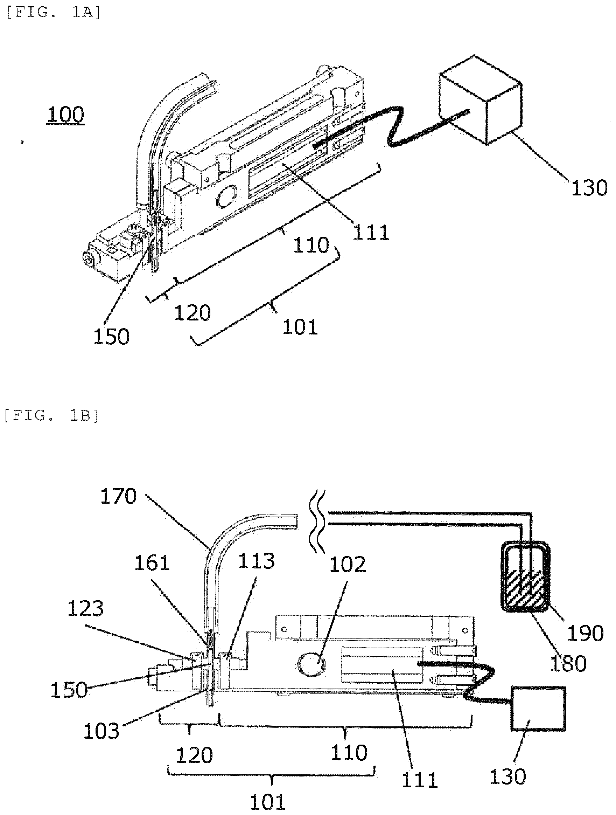

Fig. 1A is a schematic view showing an internal structure of a liquid discharge device according to Embodiment 1 of the present invention seen from an oblique direction. In a liquid discharge device 100, a base body 101 includes a driving part 110 and a fixing part 120. The driving part 110 is a part having a driving mechanism for pressurizing a nozzle 150. The fixing part 120 is a part having a mechanism for fixing the nozzle 150.

The driving part 110 is provided with a piezoelectric element 111 generating displacement. The piezoelectric element 111 extends and contracts in synchronization with a drive pulse signal generated by an external waveform generator 130 and generates displacement. The case of using the piezoelectric element 111 will be explained below; however, mechanisms other than the piezoelectric element 111 may be used as long as the mechanism generates displacement.

Fig. 1B is a schematic view showing an internal structure of the liquid discharge device 100 according to Embodiment 1 of the present invention as seen from a side direction. The driving part 110 is fixed in a rear part of the base body 101. The driving part 110 is held by a fixing bar 102 so as to hold a position of the driving part 110. The driving part 110 includes a hole into which the fixing bar 102 is inserted. The hole is processed in an oval shape in which a long diameter corresponds to a displacement direction of the piezoelectric element 111. Accordingly, the displacement of the piezoelectric element 111 is transmitted to a front direction.

Here, a pin-shaped or protruding first holding part 113 is provided at an end part of the driving part 110. The first holding part 113 moves with the driving part 110.

A second holding part 123 having a similar shape to the first holding part 113 is also provided at an end part of the fixing part 120. The second holding part 123 is held by the fixing part 120 and does not move.

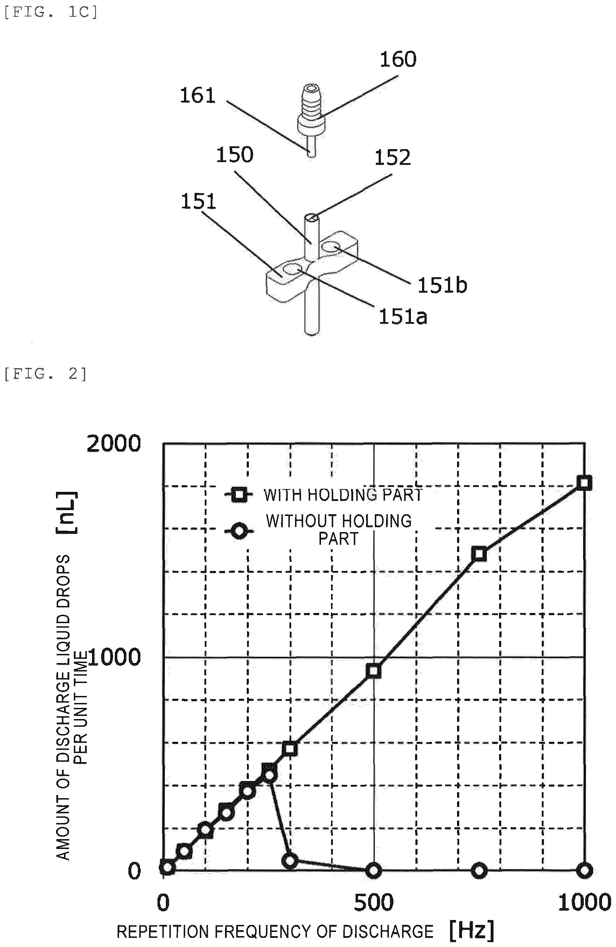

An insertion hole 103 is provided between the first holding part 113 and the second holding part 123, and the nozzle 150 is inserted thereinto. Fig. 1C is a perspective view showing the nozzle 150 according to Embodiment 1 of the present invention. A pair of held parts 151 (a first held part 151a and a second held part 151b) are provided at positions of 180 degrees in a radial direction of the nozzle 150. The held parts 151 are opened parts and are fitted to the protruding first holding part 113 and the second holding part 123. The opened parts are formed slightly smaller than the protrusions. Respective held parts 151 are fitted to the first holding part 113 and the second holding part 123. As assembled by a fitting method, positions of the nozzle 150 in a horizontal direction and a vertical direction can be prescribed with good reproducibility.

The first holding part 113 and the second holding part 123 are processed so that a diameter is increased toward a tip end thereof. This is for preventing the held parts 151 of the nozzle 150 from coming off from the first holding part 113 and the second holding part 123 and preventing the nozzle 150 from falling off due to the displacement of the first holding part 113 in the driving part 110 by the piezoelectric element 111.

Moreover, an opening 152 is formed at an upper part of the nozzle 150. A throttle 160 is inserted into the opening 152. The inside of the throttle 160 is hollow. An insertion side of the throttle 160 to the nozzle 150 is thin, and an orifice 161 is formed. An opposite side of the throttle 160 is processed in a barb joint, to which a tube 170 can be inserted and fixed. Furthermore, the tube 170 is connected to a tank 180.

### <Supply of Chemical Liquid>

When the tank 180 is pressurized, a chemical liquid 190 can be pressure-fed from the tank 180 to the tube 170. The inside of the throttle 160 and the nozzle 150 can be filled with the chemical liquid 190 through the tube 170. Accordingly, the entire liquid discharge device 100 is filled with the chemical liquid and preparation for discharge is completed.

### <Discharge Operation>

Here, when a discharge signal is transmitted from the waveform generator 130 for driving the piezoelectric element 111, the piezoelectric element 111 performs displacement corresponding to a waveform of the discharge signal. At this time, when a positive voltage is applied to the discharge signal, the piezoelectric element 111 performs displacement in an extending direction. The displacement is transmitted to the first holding part 113 through the driving part 110, applying an operation of compressing the nozzle 150 from a side direction. A pseudo closed space with a high flow-path resistance is formed by a tip end part of the nozzle 150 and the orifice 161 of the throttle 160, which functions as a pressure chamber for increasing an internal pressure.

Therefore, the displacement of compression from the side direction applied to the nozzle 150 leads to discharge of the chemical liquid 190 from the tip end part of the nozzle 150 with the pressure increase inside the pressure chamber.

Next, when a predetermined period of time passes, an output of the waveform generator 130 is reduced. At this time, the piezoelectric element 111 is returned to the original state from the extended state, namely, operation is shifted from extension to contraction in accordance with the decrease in the voltage to be applied. In the nozzle 150, this operation corresponds to a shift from compression to expansion.

Normally, when released from the compression, a period of time during which expansion is proceeding from the compressed state depends on a modulus of elasticity. Polypropylene as a resin material used for the nozzle 150 according to the embodiment has 1.12 to 1.58 GPa in the modulus of elasticity, and an autonomous expanding time experimentally requires 50 milliseconds. On the other hand, the piezoelectric element 111 shows a reaction corresponding to the voltage. The expanding time is approximately 50 microseconds though constrained by the shape, and reaction is sufficiently high as compared with an autonomous recovery time of polypropylene.

Here, the held parts 151 of the nozzle 150 are held by the first holding part 113 and the second holding part 123, thereby controlling the expanding time of the nozzle 150 more actively. That is, the expanding time of the nozzle 150 can be close to a contraction time of the piezoelectric element 111.

This means that compression/expansion of the nozzle 150 can be performed at a high repetition frequency. That is, the discharge operation can be performed at a high frequency.

It is preferable that the first holding part 113 and the second holding part 123 face each other across the nozzle 150. They are preferably arranged point-symmetrically across the nozzle 150.

Fig. 2 shows a relationship between an amount of discharge liquid drops per unit time and a repetition frequency of discharge. In a case where the held parts 151 are not provided, the linearity of the amount of discharge liquid drops per second with respect to the repetition frequency, namely, the repetition frequency of discharge is gradually reduced from a point exceeding a repetition frequency of 200 Hz, reaching a peak in the vicinity of 250 Hz and rapidly reduced afterwards, then, malfunction of discharge itself occurs.

This is caused by the following reasons. The nozzle 150 is not capable of following the driving part 110, and the driving part 110 is retracted after the nozzle 150 is compressed. As a result, a next compression operation is performed before the expansion of the nozzle 150 occurs. Accordingly, the nozzle 150 is not capable of expanding and the compressed state is kept. Consequently, pressure increase inside the nozzle 150 because discharge is not obtained.

However, in the nozzle 150 having the held parts 151 and the liquid discharge device 100 according to the present embodiment, the discharge can be maintained even at 1000 Hz though there are variations in the amount of discharge liquid drops.

Furthermore, to fix the held parts 151 of the nozzle 150 by the first holding part 113 and the second holding part 123 secures a permanent contact even at a high repetition frequency. This leads to a long lifetime of the nozzle 150.

When the contact is not secured, the expansion of the nozzle 150 does not follow the retraction of the first holding part 113 due to contraction of the piezoelectric element 111, and it is difficult to maintain the contact.

This case means that the first holding part 113 collides with the nozzle 150 when the piezoelectric element 111 extends again. The collision causes abrasion of the nozzle 150 and resultantly reduces a compression distance, which reduces the pressure and largely hinders stable discharge to reduce the lifetime of the nozzle 150.

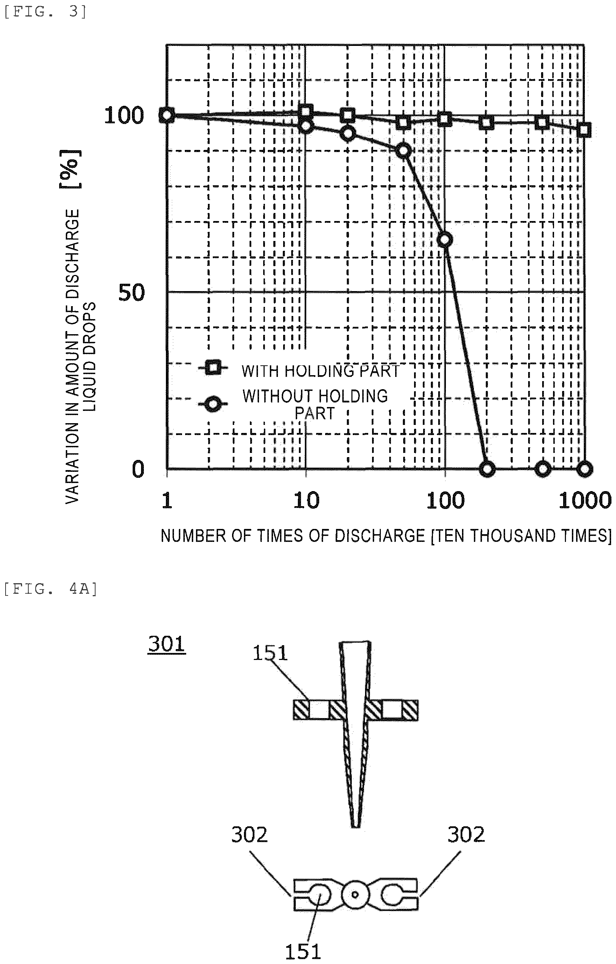

Fig. 3 is a graph showing a relationship between the amount of discharge liquid drops and the number of times of discharge. When the held parts 151 are not provided, that is, when separation between the driving part 110 and the nozzle 150 is repeated, the amount of discharge liquid drops is gradually reduced from an initial state to approximately 10% after being discharged 500 thousand times.

The reduction in the amount of discharge liquid drops is caused because the nozzle 150 is worn out slightly by repeating the separation between the driving part 110 and the nozzle 150 and the displacement of the driving part 110 is not positively transmitted to the inside of the nozzle 150. However, the reduction in the amount of discharge liquid drops is not recognized even after being discharged 10, 000, 000 times according to the nozzle provided with the held parts 151 and the liquid discharge device 100 according to the embodiment.

### <Modification Examples of Nozzle 150>

The held parts 151 have a function of transmitting the movement of the first holding part 113 and the second holding part 123 to the nozzle 150 positively. However, frequent replacement of chemical liquids may occur in various applications; therefore, easy removal of the nozzle 150 is also an important element.

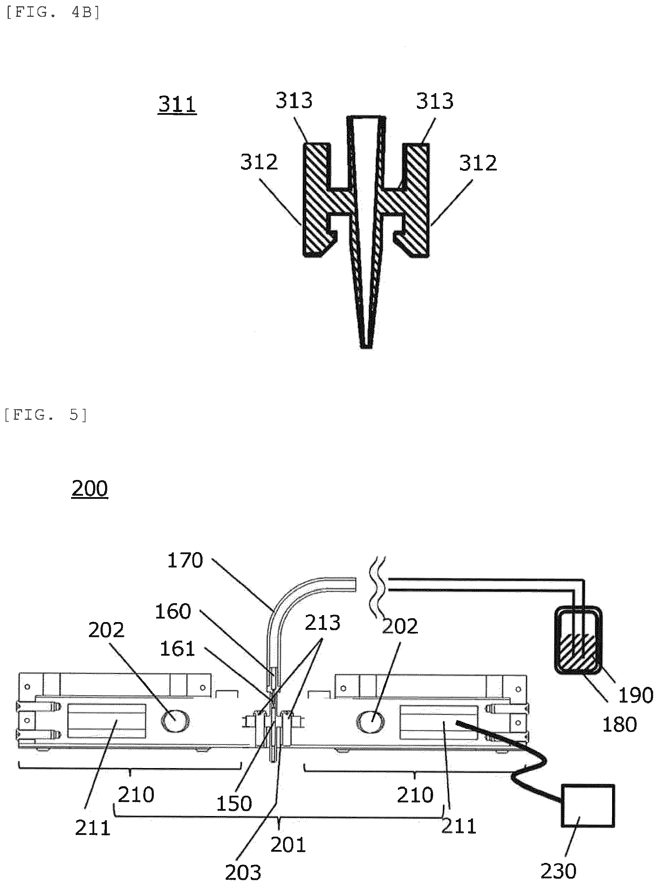

Fig. 4A shows a modification example of the nozzle 150 explained in Embodiment 1. An upper view is a cross-sectional view and a lower view is a top view. Fig. 4B is a cross-sectional view showing the modification example explained in Embodiment 1.

In a nozzle 301 shown in Fig. 4A, cutouts 302 are provided in parts of the held parts 151. Insertion of the nozzle 301, particularly, removal of the nozzle 301 can be performed with a smaller force due to the cutouts 302. It is sufficient that the cutout 302 is formed in at least one of the held parts 151.

Fig. 4B shows a side view of a nozzle 311. Hooks 312 for connecting the driving part 110 and the fixing part 120 are formed in a direction parallel to an insertion direction of the nozzle 311. As deformation parts 313 of the hooks 312 bend, insertion, particularly, removal can be easily performed. In this case, recessed parts for receiving the hooks 312 are necessary in the first holding part 113 and the second holding part 123. As deformation parts 323 of hooks 322 bend, the insertion, particularly, removal can be easily performed in the same manner as the case of the nozzle 311. Moreover, the nozzle can be manufactured at low cost as the structure becomes easy.

It is desirable that the modulus of elasticity of the nozzle is low for obtaining deformation properties. Although the state in which the nozzle is formed by a single material is shown, it is preferable to increase the modulus of elasticity on the other hand for elongating the lifetime of the nozzle; therefore, it is effective to adopt a two-stage molding of different materials or a method in which respective materials are formed separately to be integrated afterwards by adhesion.

### (Embodiment 2)

### <Structure>

Fig. 5 is a schematic view showing a cross-sectional structure of a liquid discharge device 200 according to Embodiment 2 seen from a side surface. Items not explained are the same as those of Embodiment 1. Embodiment 2 differs from Embodiment 1 in a point that the nozzle is pressurized and compressed from both sides.

In the liquid discharge device 200, a pair of driving parts 210 are mounted inside a base body 201.

Piezoelectric elements 211 generating displacement are installed in the driving parts 210. These elements expand and contract in synchronization with a drive pulse signal generated by an external waveform generator 230 and generate displacement.

The driving parts 210 are fixed at end parts of the base body 201. The driving parts 210 are held by fixing bars 202 so as to hold positions of the driving parts 210. The driving parts 210 include holes into which the fixing bars 202 are inserted. The holes are processed in the oval shape in which long diameters correspond to displacement directions of the piezoelectric elements 211. Accordingly, the displacement of the piezoelectric elements 111 is transmitted to a central direction of the base body 201.

Here, first holding parts 213 are provided at end parts of the driving parts 210. An insertion hole 203 is provided between these pair of first holding parts 213, and the nozzle 150 is inserted therein.

Moreover, the orifice 161 of the throttle 160 is inserted into the nozzle 150. The nozzle 150 is connected to the tank 180 from the throttle 160 through the tube 170.

### <Supply of Chemical Liquid>

When the tank 180 is pressurized, the inside of the throttle 160 and the nozzle 150 can be filled with the chemical liquid 190 through the tube 170. Accordingly, the entire liquid discharge device 200 is filled with the chemical liquid 190 and preparation for discharge is completed.

### <Discharge Operation>

Here, when a discharge signal is transmitted from the waveform generator 130 for driving the piezoelectric elements 211, the piezoelectric elements 211 perform displacement corresponding to a waveform of the discharge signal. At this time, when a positive voltage is applied to the discharge signal, the piezoelectric elements 211 perform displacement in the extending direction. The displacement is transmitted to the first holding parts 213 through the driving parts 210, applying an operation of compressing the nozzle 150 from both sides. A pseudo closed space with a high flow-path resistance is formed by a tip end part of the nozzle 150 and the orifice 161 of the throttle 160, which functions as a pressure chamber for increasing an internal pressure. Therefore, the displacement of compression from the side directions applied to the nozzle 150 leads to discharge of the chemical liquid 190 from the tip end part of the nozzle 150 with the pressure increase inside the pressure chamber.

As the nozzle 150 is compressed from both sides of the pair of driving parts 210 according to the embodiment, driving can be performed with apparently twice as much displacement. Accordingly, it is possible to discharge a larger amount of liquid drops than that of the liquid discharge device 100 shown in Embodiment 1, though increased in size.

Furthermore, an apparatus for applying various types of liquids can be realized by using the above liquid discharge device 200. It is possible to move an object to be applied or to move the liquid discharge device itself. The apparatus can be configured as an application apparatus having a driving part and a controller.

### <Advantages>

In the liquid discharge device according to Embodiment 2, the held parts 151 are provided in the nozzle 150 discharging the liquid to thereby constantly secure the contact between the driving parts 210 and the nozzle 150 and to transmit the operation of the driving parts 210 to the nozzle 150 positively. Therefore, the discharge can be performed at a high repetition frequency as the liquid discharge device capable of replacing the nozzle 150, and further, the lifetime of the nozzle 150 can be increased.

### (Embodiment 3)

Embodiment 3 shows application examples of the above liquid discharge devices 100, 200 and the following liquid discharge devices 700, 900. These devices can apply a minute amount of liquid with high accuracy in a certain amount and for a long period of time.

Accordingly, the devices can be utilized for applying blood to a bio-sensor or a biochip. For example, when blood is applied to a blood sensor, liquid dropping is performed by the liquid discharge device 100, 200, 700 or 900, and the blood sensor is read by a detector to find a blood value. In this method, the liquid amount of blood is accurate and a precise result can be obtained.

Application can be performed not only to the blood value sensor but also immunosensors such as a tumor marker and a heart disease marker. Moreover, the sensors may be DNA sensors for epigenetics and infectious diseases. Biosensors can be widely used as sensors.

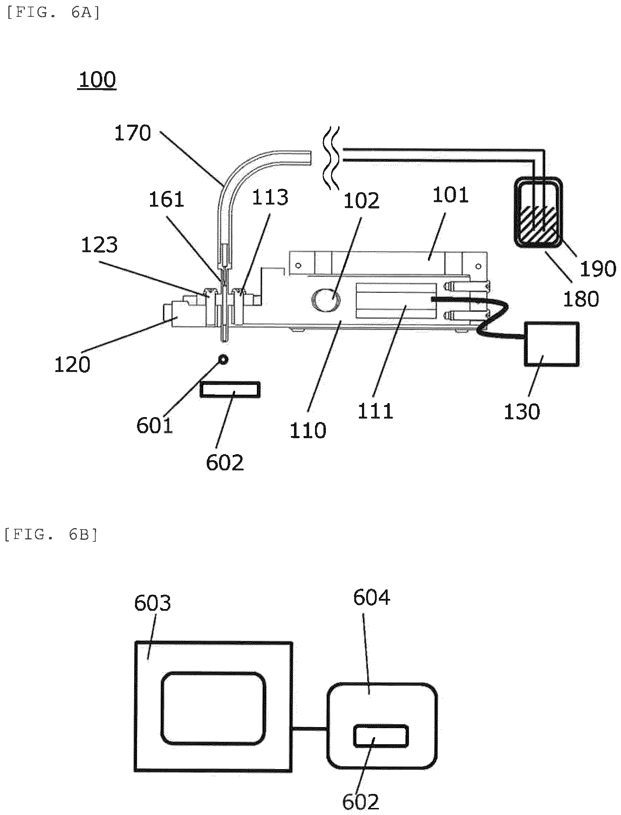

A device according to Embodiment 3 will be explained with reference to Fig. 6A and Fig. 6B. Items not explained are the same as those of Embodiments 1, 2. The liquid discharge device 100 is used for explanation; however, another liquid discharge device according to another embodiment may be used.

Fig. 6A is a side view of the liquid discharge device 100. Blood as a liquid 601 is discharged to a sensor 602 by the liquid discharge device 100.

After that, the sensor 602 is analyzed by an inspection unit shown in Fig. 6B. Fig. 6B is a front view of the inspection unit.

The sensor 602 is inserted into a detector 604 shown in Fig. 6B. The detector 604 is controlled by a controller 603, inspecting the sensor 602 and displaying a result.

Fig. 6A and Fig. 6B show an inspection device.

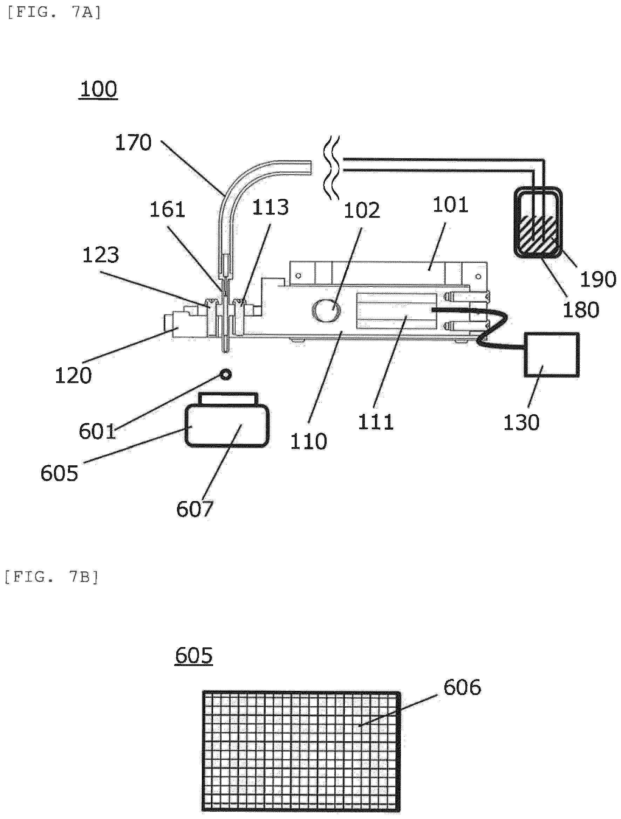

Here, it is possible to use a container 605 shown in Fig. 7B instead of using the sensor 602. Fig. 7A and Fig. 7B show an example of a cell culture device. Fig. 7B is a plan view of the container 605. Fig. 7A is a side view of the cell culture device. The container 605 has a plurality of recessed parts 606. When cells are held in respective plural recessed parts 606, and a culture solution is applied to respective plural recessed parts 606 by the liquid discharge device 100, the device can be used as the cell culture device. In this case, it is necessary to apply liquid drops to plural recessed parts 606. A stage 607 is provided at a lower part of the container 605, which can move inside a plane.

Note that there is a case where there exists one recessed part 606 in the container 605. There is also a case where liquid drops are discharged to one recessed part 606 plural times. It is possible to put a cell sheet on the recessed part 606 and to apply the liquid 601 to the cell sheet.

Moreover, various types of solutions may be applied to another sensor for manufacturing another sensor by the above device in the same manner.

### (Embodiment 4)

### <Structure>

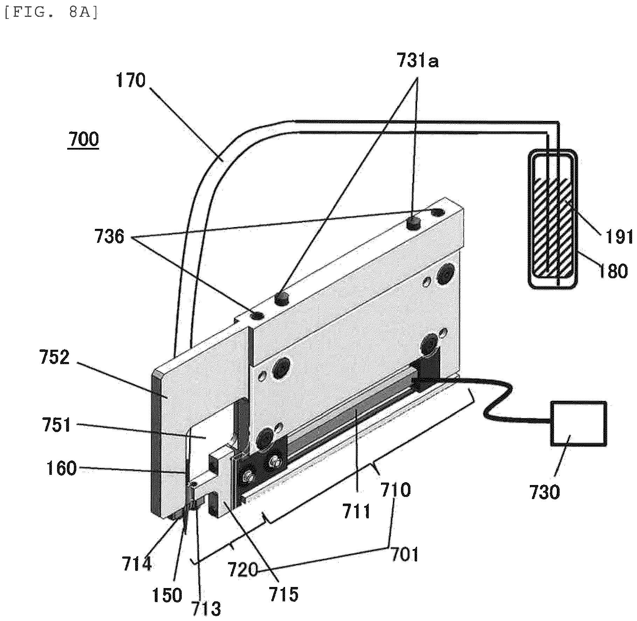

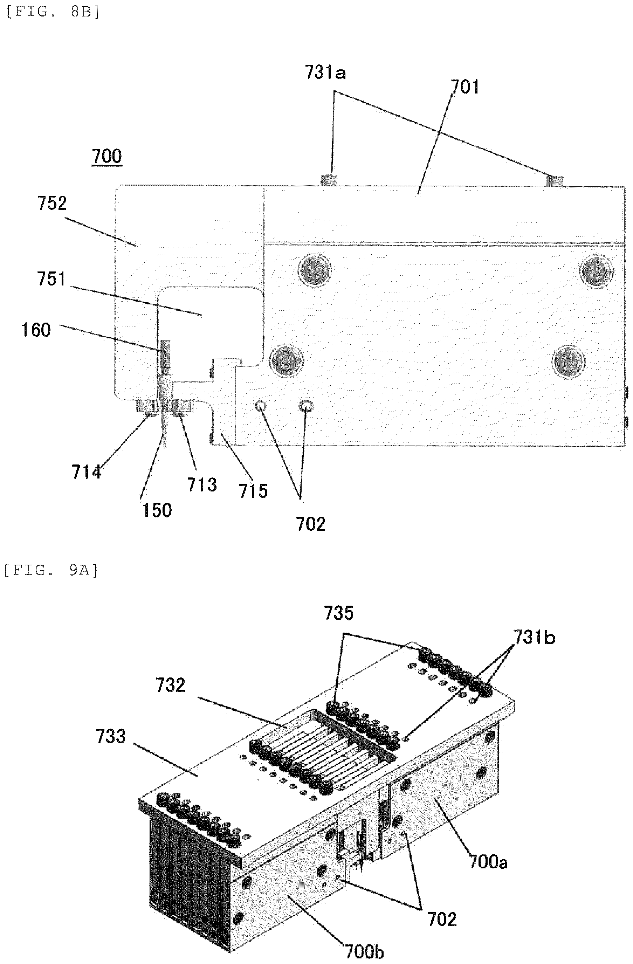

Fig. 8A is a perspective view of a liquid discharge device 700 according to Embodiment 4. Fig. 8B is a front view of the liquid discharge device 700. The liquid discharge device 700 includes a driving part 710 and a fixing part 720 in a base body 701. Items not explained are the same as those of the above embodiments.

In the driving part 710, a piezoelectric element 711 is installed as a driving mechanism generating displacement. The piezoelectric element 711 extends and contracts in synchronization with a drive pulse signal generated by an external waveform generator 730 and generates displacement.

The driving part 710 is fixed at an end part in a rear direction of the base body 701. The fixing part 720 is fixed at an end part in a front direction of the base body 701.

Through holes are formed at the end part in the front direction of the driving part 710, and a position thereof is held by fixing bars inserted into the through holes. The through holes into which the fixing bars are inserted are oval holes in which long diameters correspond to a displacement direction of the piezoelectric element 711, thereby performing transmission without impairing displacement to another direction. Each through hole is a hole regulating only one direction (up and down, or left and right).

Moreover, the displacement of the piezoelectric element 711 is transmitted to a driver 715 attached to the driving part 710. A first holding part 713 is provided at an end part of the driver 715.

On the other hand, the base body 701 is extended from above and a second holding part 714 is provided at an end part thereof.

A gap is provided between the first holding part 713 and the second holding part 714, and the nozzle 150 is inserted from below to be positioned and fixed by the first holding part 713 and the second holding part 714. For example, the nozzle 150 has a plurality of held parts 151 as shown in Fig. 4A. The plural held parts 151 correspond to the first held part 151a and the second held part 151b. Nozzles of other embodiments may be adopted.

In the case where the nozzle 150 is inserted from below, the first holding part 713 and the second holding part 714 are formed into reverse tapered pins. Alternatively, the nozzle 150 is fixed to the first holding part 713 and the second holding part 714 by fixing tools such as screws.

Furthermore, the orifice 161 of the throttle 160 is inserted into the nozzle 150. The tube 170 extending from the tank 180 is connected to the throttle 160.

Furthermore, the orifice 161 of the throttle 160 is inserted into the nozzle 150. The throttle 160 is connected to the tank 180 through the tube 170.

The structure around the nozzle 150 may adopt the above embodiments.

The liquid discharge device 700 includes an arm part 752, and the second holding part 714 is provided at an end thereof. The arm part 752 extends to an end of the liquid discharge device 700 so as to pass above the nozzle 150. The arm part 752 has an arch shape, forming an opening 751. The throttle 160 of the nozzle 150 and the tube 170 are joined by using the opening 751. Due to the arm part 752, the liquid discharge device 700 does not extend to side surface directions (a width direction) to be a compact liquid discharge device with a flat surface shape.

The nozzle 150 is attached to and detached from the liquid discharge 700 from a lower surface of the liquid discharge device 700, namely, a surface from which the liquid is discharged.

The liquid discharge device 700 has upper positioning pins 731a as a positioning mechanism on an upper surface thereof. This corresponds to the item for positioning the liquid discharge device 700 to a third object, namely, the positioning mechanism.

### <Supply of Chemical Liquid>

The tank 180 is pressurized to thereby fill the inside of the nozzle 150 with a chemical liquid 191 through the tube 170 and the throttle 160. Accordingly, preparation for discharge is completed.

### <Discharge Operation>

When a discharge signal is transmitted from the waveform generator 730 for driving the driving part 710 (piezoelectric element 111) after the above process, the piezoelectric element 711 performs displacement corresponding to a waveform of the discharge signal. At this time, when a positive voltage is applied to the discharge signal, the piezoelectric element 711 performs displacement in the extending direction. The displacement is transmitted to the first holding part 713 through the driving part 710, applying an operation of compressing the nozzle 150 from both sides. A pseudo closed space with a high flow-path resistance is formed by the tip end part of the nozzle 150 and the orifice 161 of the throttle 160, which functions as a pressure chamber for increasing an internal pressure. Therefore, the displacement of compression from side directions applied to the nozzle 150 leads to discharge of the chemical liquid 191 from the tip end part of the nozzle 150 with the pressure increase inside the pressure chamber.

According to the above embodiment, the base body 701 is formed in the same plane with the driving part 710, the second holding part 714 and the first holding part 713 respectively (namely, in a thin plate-shaped body, in a thin rectangular-parallelepiped shape).

Accordingly, the operation of the piezoelectric element 711 can be performed in a state of being enclosed in the base body 701. Therefore, the liquid discharge device 700 can complete the discharge operation independently without requiring other components.

As a result, it is possible to add a plurality of base bodies 701 easily, and a structural neighboring interference (crosstalk) can be reduced to the utmost limit as respective base bodies 701 are independent. Accordingly, a multi-type liquid discharge device capable of stably performing discharge can be configured easily.

Furthermore, an apparatus for applying various types of liquids can be realized by using the above liquid discharge device. It is possible to move an obj ect to be applied or to move the liquid discharge device itself. The apparatus can be configured as an application apparatus having a driving mechanism and a control mechanism.

### <Plural Liquid Discharge Devices 700>

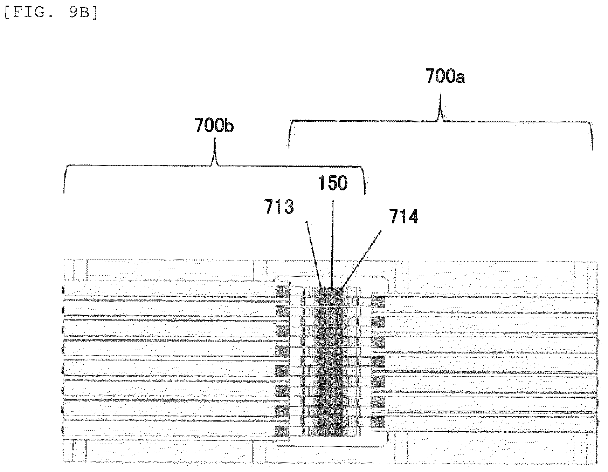

A liquid discharge device 900 configured by combining plural liquid discharge devices 700 is shown in Fig. 9A and Fig. 9B. Fig. 9A is a perspective view of the liquid discharge device 900. Fig. 9B is a bottom view of the liquid discharge device 900.

A block 700a having plural liquid discharge devices 700 and a block 700b having plural liquid discharge devices 700 are arranged on one plate 733.

The upper positioning pins 731a of respective liquid discharge devices 700 are fitted to positioning pin holes 731b on the plate 733 to be positioned. Upper fixing part screws 735 are fitted to fixing parts 736 of the liquid discharge devices 700 through an opening on an upper surface of the plate 733, and the plate 733 is fixed to the respective liquid discharge devices 700 independently.

On the other hand, bars of fixing parts 702 are inserted into through holes provided in the respective liquid discharge devices 700 in a horizontal direction. As a result, the respective liquid discharge devices 700 are fixed.

The respective liquid discharge devices 700 are not directly joined (contacted) . Rather, they are joined through the fixing bars 702 or through the plate 733. Accordingly, vibration of the liquid discharge devices 700 due to respective driving is not easily transmitted to adjacent other liquid discharge devices 700. As a result, discharge of the respective discharge devices 700 are not easily affected one another.

As shown in Fig. 8A, places where the nozzle 150 is held (parts of the first holding part 713, the second holding part 714, the fixing part 720, and the arm part 752) are thinner than the base body 701 (a part where the driving part 710 is positioned). Accordingly, respective nozzles 150 are not contacted. At least the place of the second holding part 714 is thinner than the base body 701.

An opening 732 is a through hole into which the tube 170 is inserted. In Fig. 9A, the tube 170 is not shown. The nozzle 150 is attached from a lower part of the liquid discharge device 700.

Although the tube 170 is not shown in Fig. 9A, the arm part 752 (Fig. 8A) has a thickness half of the thickness of the base body 701, and the tube 170 can be inserted from a gap between adjacent arm parts 752.

In Fig. 9A, the plural liquid discharge devices 700 are integrated by the fixing bars 702 and the plate 733 in respective side surface directions in the block 700a to thereby form one block. Also in the block 700b, the plural liquid discharged devices 700 are integrated by the fixing bars 702 and the plate 733 in the side surface directions to thereby form one block. The block 700a and the block 700b are arranged so as to face each other.

However, the arm parts 752 of respective liquid discharge devices 700 are alternately inserted, respectively. As a result, portions of respective nozzles 150 are arranged so as to be alternately aligned. The nozzles 150 are arranged at high density.

Note that the device can be used as the liquid discharge device by providing only the block 700a.

### (As a Whole)

Embodiments 1 to 4 can be combined.

The relationship between the held part 151 and the first holding part 113/the second holding part 123 may be reverse. The held parts 151 may have the pin shape or the protruding shape and the first holding part 113 and the second holding part 123 may be openings or holes. It is sufficient that they form a fitting mechanism.

At least one of the two held parts 151 (the first held part 151a, the second held part 151n) may be held to the driving part. Moreover, it is preferable that the other is held in the fixing part or the driving part.

The held parts 151 are not fundamental, and it is preferable that a place capable of being fixed to another member is provided in the nozzle.

### [INDUSTRIAL APPLICABILITY]

The liquid discharge device can be suitably used as the liquid discharge device used for supplying or dispensing the chemical liquid, the reagent and so on in fields of drug discovery, medical treatment, biotechnology and the like, in which high reliability is required.

### [REFERENCE SIGNS LIST]

- 100: LIQUID DISCHARGE DEVICE
- 101: BASE BODY
- 102: FIXING BAR
- 103: INSERTION HOLE
- 110: DRIVING PART
- 111: PIEZOELECTRIC ELEMENT
- 113: FIRST HOLDING PART
- 120: FIXING PART
- 123: SECOND HOLDING PART
- 130: WAVEFORM GENERATOR
- 150: NOZZLE
- 151: HELD PART
- 151a: FIRST HELD PART
- 151b: SECOND HELD PART
- 152: OPENING
- 160: THROTTLE
- 161: ORIFICE
- 170: TUBE
- 180: TANK
- 190: CHEMICAL LIQUID
- 191: CHEMICAL LIQUID
- 200: LIQUID DISCHARGE DEVICE
- 201: BASE BODY
- 202: FIXING BAR
- 203: INSERTION HOLE
- 210: DRIVING PART
- 211: PIEZOELECTRIC ELEMENT
- 213: FIRST HOLDING PART
- 230: WAVEFORM GENERATOR
- 301: NOZZLE
- 302: CUTOUT
- 311: NOZZLE
- 312: HOOK
- 313: DEFORMATION PART
- 322: HOOK
- 323: DEFORMATION PART
- 601: LIQUID
- 602: SENSOR
- 603: CONTROLLER
- 604: DETECTOR
- 605: CONTAINER
- 606: RECESSED PART
- 607: STAGE
- 700: LIQUID DISCHARGE DEVICE
- 700a: BLOCK
- 700b: BLOCK
- 701: BASE BODY
- 702: FIXING BAR
- 710: DRIVING PART
- 711: PIEZOELECTRIC ELEMENT
- 713: FIRST HOLDING PART
- 714: SECOND HOLDING PART
- 715: DRIVER
- 720: FIXING PART
- 730: WAVEFORM GENERATOR
- 731a: PIN
- 731b: PIN HOLE
- 732: OPENING
- 733: PLATE
- 735: UPPER FIXING PART SCREW
- 736: FIXING PART
- 751: OPENING
- 752: ARM PART
- 900: LIQUID DISCHARGE DEVICE

## Claims

1. A liquid discharge device comprising:
a nozzle from which liquid drops are discharged;
a fixing part for fixing the nozzle; and
a driving part generating displacement,
wherein the nozzle is fixed to a first holding part in the driving part and a second holding part in the fixing part.

2. The liquid discharge device according to claim 1,
wherein the first holding part and the nozzle as well as the second holding part and the nozzle are joined by a fitting method.

3. The liquid discharge device according to claim 1,
wherein a thickness of the fixing part is thinner than a thickness of the driving part.

4. The liquid discharge device according to claim 1,
wherein the nozzle is combined with the first holding part and the second holding part at a lower surface of the liquid discharge device.

5. The liquid discharge device according to claim 1,
wherein the second holding part is provided at an arm part passing above the nozzle.

6. The liquid discharge device according to claim 1,
wherein the arm part forms an opening.

7. The liquid discharge device according to claim 1,
wherein a positioning mechanism for attaching the liquid discharge device is provided on an upper surface of the liquid discharge device.

8. The liquid discharge device according to claim 1,
wherein the second holding part, the driving mechanism, and the first holding part are formed in the same plane.

9. The liquid discharge device according to claim 1,
wherein the driving mechanism is a piezoelectric element.

10. A liquid discharge device comprising:
a plurality of liquid discharge devices according to claim 1,
wherein the plural liquid discharge devices are arranged on one plate at respective upper surfaces thereof.

11. The liquid discharge device according to claim 10,
wherein the plural liquid discharge devices have plural through holes respectively and are fixed by inserting fixing bars into the plural through holes.

12. The liquid discharge device according to claim 10,
wherein the plural liquid discharge devices form two blocks integrated on respective side surfaces thereof, and
the two blocks are arranged so as to face each other.

13. The liquid discharge device according to claim 12,
wherein the two blocks are configured so that fixing parts of respective plural liquid discharge devices are adjacent to one another and alternately positioned.

14. A sensor manufacturing device comprising:
the liquid discharge device according to claim 1;
a sensor to which liquid is applied from the liquid discharge device;
a detector detecting the sensor; and
a controller controlling the detector.

15. A cell culture device comprising:
the liquid discharge device according to claim 1;
a container to which liquid is applied from the liquid discharge device; and
a stage moving the container.
